**Europäisches Patentamt**

**European Patent Office**

**Office européen des brevets**

(19)

(11) **EP 0 864 358 A1**

(12) **EUROPÄISCHE PATENTANMELDUNG**

(43) Veröffentlichungstag:
16.09.1998 Patentblatt 1998/38

(51) Int. Cl.⁶: **B01J 23/38**, B01J 23/42,
C07C 51/377

(21) Anmeldenummer: 98102864.0

(22) Anmeldetag: 19.02.1998

(84) Benannte Vertragsstaaten:
AT BE CH DE DK ES FI FR GB GR IE IT LI LU MC
NL PT SE
Benannte Erstreckungsstaaten:
AL LT LV MK RO SI

(30) Priorität: 13.03.1997 DE 19710376

(71) Anmelder: **Clariant GmbH**
**65929 Frankfurt am Main (DE)**

(72) Erfinder:
• **Seidel, Andreas, Dr.**
**50999 Köln (DE)**
• **Pottkämper, Karen**
**50354 Hürth (DE)**

(54) **Edelmetallhaltiger Trägerkatalysator zur hydrierenden Enthalogenierung von alpha-halogenierten Carbonsäuren sowie ein Verfahren zu seiner Herstellung**

(57)     Die Erfindung betrifft Suspensionsträgerkatalysator zur hydrierenden Dehalogenierung von α-halogenierten Carbonsäuren mit hochdisperser, homogener Edelmetalldotierung mit einem Edelmetall der VIII. Nebengruppe des Periodensystems, erhalten durch Imprägnierung eines trockenen porösen Trägermaterials mit einer Lösung eines Edelmetallsalzes und anschließende Reduktion des vom Trägermaterial aufgenommenen Edelmetallsalzes zum reinen Edelmetall mittels Wasserstoff, dadurch gekennzeichnet, daß der Suspensionsträgerkatalysator einen Edelmetallgehalt zwischen 0,5 und 15 Gew.-% aufweist, sowie ein Verfahren zu seiner Herstellung.

EP 0 864 358 A1

**Beschreibung**

Die Erfindung betrifft einen Suspensionsträgerkatalysator zur hydrierenden Dehalogenierung von $\alpha$-halogenierten Carbonsäuren mit hochdisperser, homogener Edelmetalldotierung mit einem Edelmetall der VIII. Nebengruppe des Periodensystems, erhalten durch Imprägnierung eines trockenen porösen Trägermaterials mit einer Lösung eines Edelmetallsalzes und anschließende Reduktion des vom Trägermaterial aufgenommenen Edelmetallsalzes zum reinen Edelmetall mittels Wasserstoff, ein Verfahren zu seiner Herstellung sowie die Verwendung des nach dem erfindungsgemäßen Verfahren hergestellten Suspensionsträgerkatalysators.

Bei der Herstellung von Monochloressigsäure durch direkte Chlorierung von Essigsäure mit Chlorgas entstehen als unerwünschte Nebenprodukte Dichloressigsäure und in Spuren Trichloressigsäure. Aus dem Stand der Technik sind eine Reihe von Verfahren bekannt, in denen beschrieben wird, die Gehalte an den vorgenannten Nebenprodukten, insbesondere von Dichloressigsaure, durch eine geeignete Nachbehandlung des Rohproduktes zu reduzieren.

Ein technisch weit verbreitetes Verfahren zur Entfernung von Dichloressigsäure aus Monochloressigsaure ist die hydrierende Enthalogenierung, die durch Umsetzung mit Wasserstoff in Gegenwart eines Katalysators erfolgt:

$$Cl_nH_{3-n}C\text{-}COOH + H_2 \xrightarrow{\text{Katalysator}} Cl_{n-l}H_{4-n}C\text{-}COOH + HCl \; (n = 1 \text{ bis } 3)$$

Da die Reaktionsgeschwindigkeit von Monochloressigsäure (n = 1) in der Hydrierung erheblich kleiner ist als die von der Dichloressigsäure (n = 2) ist dieses Verfahren mit nur geringen Verlusten an Monochloressigsäure durchführbar.

Für dieses Verfahren sind insbesondere Trägerkatalysatoren geeignet, die mit einem Metall aus der VIII. Nebengruppe des Periodensystems dotiert sind. Hierbei ist Palladium bevorzugt.

Die DE-PS 22 40 466 beschreibt ein Verfahren zur Herstellung eines aus einem feinkörnigen Trägermaterial und Palladiummetall bestehenden Katalysators zur partiellen Dehalogenierung von Di- und/oder Trichloressigsäure in Gegenwart von Wasserstoff unter Bildung von Monochloressigsäure, durch Tränken des trockenen porösen Trägermaterials mit einer Lösung eines Palladiumsalzes und anschließende Reduktion des vom Trägermaterial aufgenommenen Palladiumsalzes zum Palladiummetall mit Hilfe eines geeigneten Reduktionsmittels, bei dem man zur Anreicherung des Palladiummetalls ausschließlich auf der Oberfläche des speziellen feinkörnigen Trägermaterials dieses in trockenem Zustand mit dem reinen Lösemittel für das Palladiumsalz bis zu einem Sättigungsgrad von 50 bis 80 % tränkt, und dann das derart vorbehandelte Trägermaterial mit einer zur vollständigen Sättigung ausreichenden Menge an Palladiumsalzlösung imprägniert und schließlich das auf den Träger aufgebrachte Palladiumsalz in bekannter Weise zu Palladium reduziert.

Bei diesem Verfahren wird demnach erst das Lösemittel (Wasser) und danach die Palladiumchloridlösung aufgebracht. Hierbei können aber nur relativ geringe Gehalte an Palldium auf das Trägermaterial erreicht werden, da es beim Einsatz von größeren Palladiumchlorid-Mengen und anschließender Reduzierung mittels Hydrazin-Lösung zu empfindlichen Verlusten an Palladium kommt.

Auch die anderen bekannten Verfahren zur Herstellung von geeigneten Katalysatoren weisen jedoch den Nachteil auf, daß es nicht möglich ist, Katalysatoren mit höheren Edelmetallgehalten zu erhalten, die bei Dauerbelastung eine hohe Stabilität aufweisen.

Es ist daher Aufgabe der Erfindung, einen Suspensionsträgerkatalysator zur Verfügung zu stellen, der einen hohen Edelmetallgehalt, eine lange Standzeit und eine zum hohen Edelmetallgehalt proportionale katalytische Aktivität aufweist.

Diese Aufgabe wird gelöst durch einen Suspensionsträgerkatalysator der eingangs beschriebenen Art, dadurch gekennzeichnet, daß der Suspensionsträgerkatalysator einen Edelmetallgehalt zwischen 0,5 und 15 Gew.-% aufweist.

Bevorzugt weist der Suspensionsträgerkatalysator einen Edelmetallgehalt von 5 bis 10 Gew.-% auf.

Bevorzugt wird als Edelmetall Platin eingesetzt.

Ebenfalls bevorzugt wird als Edelmetall Palladium eingesetzt.

Bevorzugt ist beim erfindungsgemäßen Suspensionsträgerkatalysator das Edelmetall in einer mittleren Primärkristallitgröße von weniger als 30 nm hochdispers homogen über das gesamte Trägerkornvolumen verteilt.

Es ist ebenfalls Aufgabe der Erfindung, ein Verfahren zur Herstellung von Suspensionsträgerkatalysatoren zur hydrierenden Dehalogenierung von $\alpha$-halogenierten Carbonsäuren zur Verfügung zu stellen, mit dem Katalysatoren mit hohen Edelmetallgehalten hergestellt werden können, die bei Dauerbelastung eine hohe Aktivität über lange Zeit bewahren.

Diese Aufgabe wird gelöst durch ein Verfahren zur Herstellung eines Suspensionsträgerkatalysators zur hydrierenden Dehalogenierung von $\alpha$-halogenierten Carbonsäuren mit hochdisperser, homogener Edelmetalldotierung mit einem Edelmetall der VIII. Nebengruppe des Periodensystems und einem Edelmetallgehalt zwischen 0,5 und 15 Gew.-%, durch Imprägnierung eines trockenen porösen Trägermaterials mit einer Lösung eines Edelmetallsalzes und anschließende Reduktion des vom Trägermaterial aufgenommenen Edelmetalles zum reinen Edelmetall mittels eines geeigneten Reduktionsmittels, __dadurch gekennzeichnet__, daß das trockene poröse Trägermaterial mit einer Menge an Lösung des Edelmetallsalzes imprägniert wird, die der vom Porenvolumen des trockenen porösen Trägermaterials maximal absorbierbaren Menge entspricht und als Reduktionsmittel Wasserstoff eingesetzt wird.

Bevorzug wird als Edelmetall Platin eingesetzt.

Besonders bevorzugt wird das Edelmetall Palladium eingesetzt.

Bei der Lösung des Edelmetalls handelt es sich bevorzugt um eine wäßrige Lösung.

Bevorzugt wird als Trägermaterial Kieselgel ($SiO_2$) eingesetzt.

Ebenfalls bevorzugt wird als Trägermaterial Aluminiumoxid ($Al_2O_3$) eingesetzt.

Ebenfalls ist als Trägermaterial Zirkonoxid ($ZrO_2$) einsetzbar.

Bevorzugt wird das Trägermaterial in Form eines Pulvers eingesetzt.

Bevorzugt weist das Pulver eine mittlere Partikelgröße von 50 bis 200 $\mu$m, eine BET-Oberfläche zwischen 250 und 600 $m^2$/g und ein Porenvolumen von 0,6 bis 1,2 ml/g auf.

Bevorzugt wird das Trägermaterial nach der Imprägnierung einer Trocknung unterzogen, durch die absorbiertes Wasser entfernt wird.

Die Erfindung betrifft auch die Verwendung der erfindungsgemäßen Suspensionsträgerkatalysatoren oder hergestellt nach dem erfindungsgemäßen Verfahren zur Dehalogenierung von $\alpha$-halogenierten Carbonsäuren.

Bevorzugt handelt es sich bei der vorgenannten Dehalogenierung um eine De-Chlorierung.

Die Erfindung wird durch die nachstehenden Beispiele erläutert:

Beispiel 1 (Vergleich entsprechend DE-PS 22 40 466)

In einem Becherglas werden 100 g Trägermaterial (Kieselgel der Firma Grace, mittlere Korngröße etwa 120 $\mu$m) unter ständigem Umrühren zunächst mit 50 g Wasser und dann mit einer Lösung aus 45 g Palladiumdichlorid (20 Gew.-%, salzsauer in Wasser) besprüht, was einem theoretischem Palladiumgehalt von 9 Gew.-% Palladium, bezogen auf das trockene Trägermaterial, entspricht. Man erhält ein gleichmäßig orange gefärbtes Material.

Zur Reduktion wird eine Lösung aus 10 g wäßrigem Hydrazin (50 Gew.%), 16 g Ammoniak (25 Gew.-%) und 300 ml Wasser zubereitet. Der imprägnierte Träger wird portionsweise unter Rühren bei Raumtemperatur in die vorgelegte ammoniakalische Hydrazin-Lösung eingetragen, wobei sich der Fortgang der Reduktion sowohl durch Schwarzfärbung des Materials als auch Blasenbildung (durch $N_2$-Entwicklung) bemerkbar macht. Es wird 2 Stunden nachgerührt.

Danach wird der fertige Katalysator über eine Porzellannutsche abgesaugt und mit Wasser neutral gewaschen. Der noch feuchte Katalysator wird im Trockenschrank bei 100 °C bis zur Massekonstanz getrocknet. Eine Atomadsorptionsanalyse des trockenen Materials ergab ein Palladiumgehalt von 7,2 Gew.-%, was einem Verlust von 20 Gew.-% Palladium während der Herstellung des Katalysators entspricht.

Beispiel 2 (gemäß der Erfindung)

In einem 2 l-Glaskolben werden 100 g des Trägermaterials wie im Beispiel 1 vorgelegt und eine Mischung aus 35 g Palladiumdichloridlösung (20 Gew.-%, salzsauer in Wasser) und 45 g Wasser dazugegeben, was einem theoretischem Palladiumgehalt von 7 Gew.-% Palladium, bezogen auf das trockene Trägermaterial, entspricht. Zur vollständigen Benetzung des Trägers wird der Kolben 1 Stunden an einem Rotationsverdampfer mit niedriger Tourenzahl gedreht. Es resultiert ein gleichmäßig orange gefärbtes Material.

Der feuchte, aber noch fließfähige Katalysator wird in eine Porzellanschale überführt und im Trockenschrank bei 100 °C bis zur Massekonstanz getrocknet.

Zur Reduktion mit Wasserstoffwird der trockene, rötliche, imprägnierte Träger in ein vertikal gestelltes Doppelmantel-Glasrohr mit 5 cm Innendurchmesser und im Boden eingelassener Glasfritte überführt, das mit 100 °C heißem Öl über den Mantel beheizt wird, und durch die Glasfritte 10 Minuten lang mit Stickstoff gespült. Danach wird die Gaszufuhr auf 5 l/h Wasserstoffumgestellt und so lange durchgeleitet, bis der gesamte Katalysator tief schwarz gefärbt ist (ca. 15 min). Es wird noch ca. 10 Minuten nachhydriert und dann 20 Minuten mit Stickstoff gespült. Eine Atomabsorptionsanalyse des trockenen Materials ergab einen Palladiumgehalt von 7 Gew. -%.

Beispiel 3 (Anwendung)

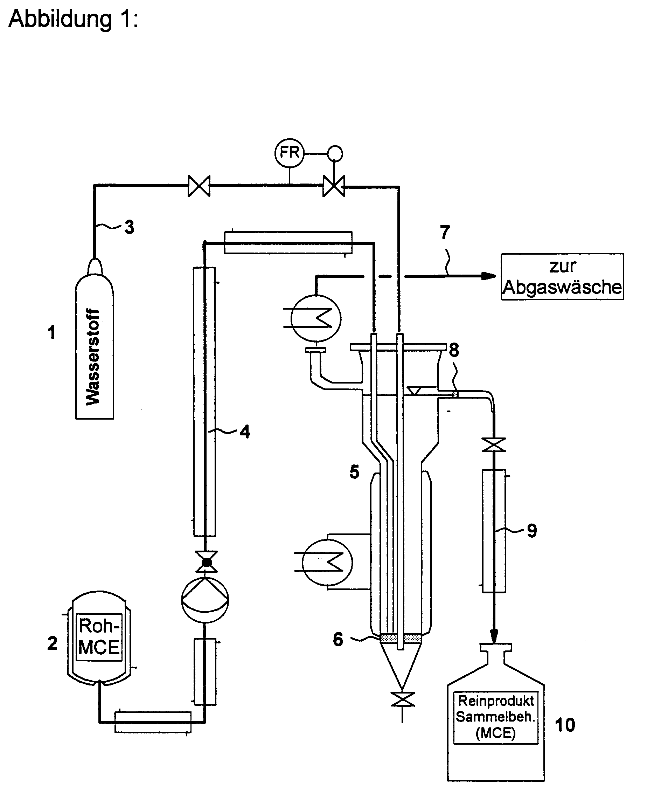

Der Reaktor 5 der in der Abbildung 1 dargestellten Versuchsapparatur wurde mit 600 ml geschmolzener roher Monochloressigsäure und 15 g getrocknetem Katalysator gemäß Tabelle 1 befüllt. Über die Mantelheizung wurde der Inhalt auf die Reaktionstemperatur von 130 °C erwärmt und die Reaktion durch geregeltes Eindosieren von 50 l Wasserstoff pro Stunde aus Behälter 1 über die Leitung 3 gestartet. Dabei wird der Wasserstoff durch ein Tauchrohr unter die in den Reaktorboden eingefaßte Porzellanfritte 6 eingespeist und zerperlt in dieser beim Aufsteigen in kleine Gasblasen. Es wurde auf kontinuierlichen Betrieb umgestellt, indem ca. 100 ml rohe aufgeschmolzene Monochloressigsäure pro Stunde aus der beheizten Vorlage 2 über die beheizte Leitung 4, die in ein bis zur Fritte 6 herabreichendes Tauchrohr mündet, in den Reaktor 5 eingepumpt wurden. In gleichem Maß, in dem der Füllstand von 5 steigt, läuft über die in den Auslauf zu 9 eingearbeitete Porzellanfritte 8 Reaktionsprodukt ab und wird über die beheizte Leitung 9 in den Produktsammelbehälter 10 geführt.

Der während der Reaktion gebildete Chlorwasserstoffwird zusammen mit nicht umgesetztem Wasserstoffüber einen Intensivkühler geführt, in dem verdampfte Anteile von Essigsäure, Monochloressigsäure und Dichloressigsäure kondensiert werden, und von dort nach 5 zurücklaufen. Aus dem Kühler werden die Gase einer Abgaswäsche zugeführt.

Zu den in der Tabelle 1 angeführten Zeiten wurden aus 9 Proben entnommen und mittels Flüssigkeitshochdruckchromatographie (HPLC) analysiert. Der Inhalt des Produktsammelbehälters 10 wurde ebenfalls analysiert.

Tabelle 1

| Verlauf kontinuierlicher Versuche | | | | | | | | | |
|---|---|---|---|---|---|---|---|---|---|
| Katalysator aus Vergleichsbeispiel | | | | | Katalysator gemäß Anmeldung | | | | |
| t [h] | Einsatz [Gew.-%] | | Produkt [Gew.-%] | | t [h] | Einsatz [Gew.-%] | | Produkt [Gew.-%] | |
| | MCE | DCE | MCE | DCE | | MCE | DCE | MCE | DCE |
| 37 | 80.80 | 3.30 | 83.44 | 0.58 | 19 | 81.70 | 3.30 | 84.12 | 0.35 |
| 94 | 81.50 | 3.40 | 81.25 | 0.57 | 35 | 81.70 | 3.30 | 84.17 | 0.37 |
| 102 | 81.50 | 3.40 | 84.91 | 0.58 | 125 | 81.30 | 3.40 | 83.25 | 0.41 |
| 118 | 81.50 | 3.40 | 84.91 | 0.63 | 133 | 81.30 | 3.40 | 84.83 | 0.43 |
| 126 | 81.50 | 3.40 | 85.43 | 0.66 | 157 | 81.30 | 3.40 | 82.62 | 0.43 |
| 134 | 81.50 | 3.40 | 85.92 | 0.66 | 181 | 81.30 | 3.40 | 83.51 | 0.38 |
| 150 | 81.50 | 3.40 | 85.53 | 0.63 | 189 | 81.30 | 3.40 | 84.50 | 0.35 |
| 166 | 81.50 | 3.40 | 85.96 | 0.65 | 205 | 81.30 | 3.40 | 84.48 | 0.41 |
| 209 | 81.60 | 3.40 | 83.48 | 0.67 | 254 | 82.70 | 3.20 | 83.61 | 0.39 |
| 217 | 81.60 | 3.40 | 83.13 | 0.70 | 278 | 82.70 | 3.20 | 85.09 | 0.40 |
| 257 | 81.60 | 3.40 | 81.13 | 0.74 | | | | | |
| 265 | 81.60 | 3.40 | 84.51 | 0.72 | | | | | |
| 273 | 81.60 | 3.40 | 84.18 | 0.80 | | | | | |
| $\Sigma^*$ | 81.35 | 3.37 | 83.42 | 0.65 | $\Sigma^*$ | 81.80 | 3.31 | 84.56 | 0.39 |

* Durchschnittsgehalte von Roh-MCE und hydriertem Produkt aus Mengenbilanz

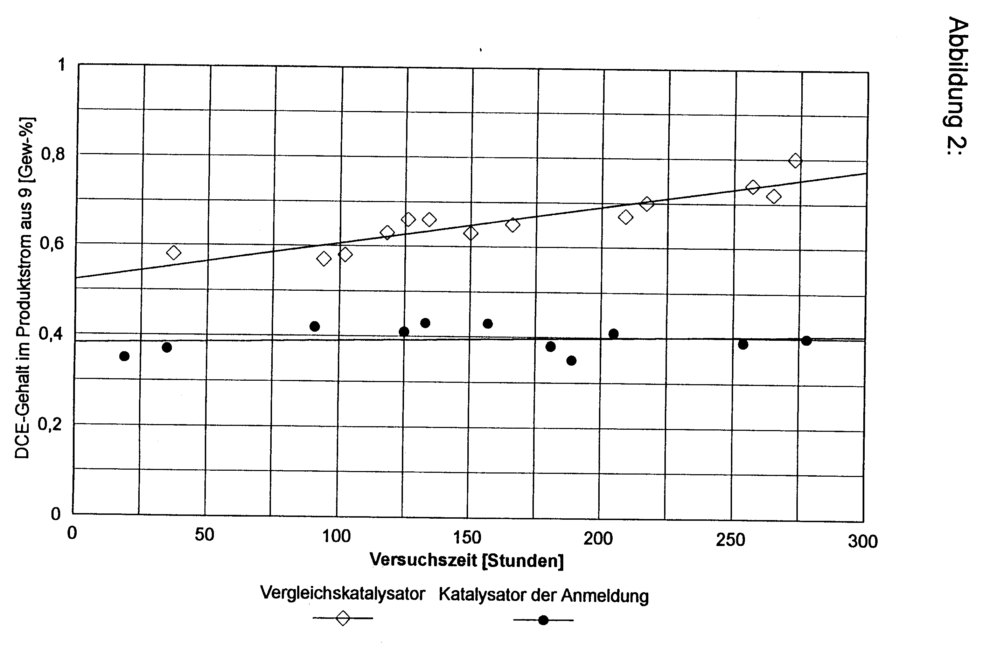

Aus den Daten der Tabelle 1 und dem graphischen Auftrag des Versuchsverlaufs (Abb. 2) ergeben sich deutliche Vorteile für den nach dem erfindungsgemäßen Verfahren hergestellten Suspensionsträgerkatalysator gegenüber Katalysatoren, wie sie nach dem Stand der Technik eingesetzt wurden. Dies äußert sich darin, daß der nach dem erfindungsgemäßen Verfahren hergestellte Suspensionträgerkatalysator

a) eine stabilere Aktivität als der Vergleichkatalysator aufweist,
b) im selben Zeitraum eine deutlich größere Menge Dichloressigsäure als der Vergleichkatalysator nach dem Stand der Technik umsetzt (88 % gegenüber 81 %),
c) im selben Zeitraum eine deutlich geringere Menge an Monochloressigsäure als der Vergleichkatalysator nach dem Stand der Technik umsetzt (Zuwachs um 2,76 Gew.-% gegenüber 2,07 Gew.-%).

**Patentansprüche**

1. Suspensionsträgerkatalysator zur hydrierenden Dehalogenierung von $\alpha$-halogenierten Carbonsäuren mit hochdisperser, homogener Edelmetalldotierung mit einem Edelmetall der VIII. Nebengruppe des Periodensystems, erhalten durch Imprägnierung eines trockenen porösen Trägermaterials mit einer Lösung eines Edelmetallsalzes und anschließende Reduktion des vom Trägermaterial aufgenommenen Edelmetallsalzes zum reinen Edelmetall mittels Wasserstoff, <u>dadurch gekennzeichnet</u>, daß der Suspensionsträgerkatalysator einen Edelmetallgehalt zwischen 0,5 und 15 Gew.-% aufweist.

2.  Suspensionsträgerkatalysator nach Anspruch 1, <u>dadurch gekennzeichnet</u>, daß er einen Edelmetallgehalt von 5 bis 10 Gew.-% aufweist.

3.  Suspensionsträgerkatalysator nach Anspruch 1 oder 2, <u>dadurch gekennzeichnet</u>, daß als Edelmetall Platin eingesetzt wird.

4.  Suspensionsträgerkatalysator nach Anspruch 1 oder 2, <u>dadurch gekennzeichnet</u>, daß als Edelmetall Palladium eingesetzt wird.

5.  Suspensionsträgerkatalysator nach einem oder mehreren der Ansprüche 1 bis 4, <u>dadurch gekennzeichnet</u>, daß das Edelmetall in einer mittleren Primärkristallitgröße von weniger als 30 nm hochdispers homogen über das gesamte Trägerkornvolumen verteilt ist

6.  Verfahren zur Herstellung eines Suspensionsträgerkatalysators zur hydrierenden Dehalogenierung von $\alpha$-halogenierten Carbonsäuren mit hochdisperser, homogener Edelmetalldotierung mit einem Edelmetall der VIII. Nebengruppe des Periodensystems und einem Edelmetallgehalt zwischen 0,5 und 15 Gew.-%, durch Imprägnierung eines trockenen porösen Trägermaterials mit einer Lösung eines Edelmetallsalzes und anschließende Reduktion des vom Trägermaterial aufgenommenen Edelmetalles zum reinen Edelmetall mittels eines geeigneten Reduktionsmittels, <u>dadurch gekennzeichnet</u>, daß das trockene poröse Tragermaterial mit einer Menge an Lösung des Edelmetallsalzes imprägniert wird, die der vom Porenvolumen des trockenen porösen Trägermaterials maximal absorbierbaren Menge entspricht und als Reduktionsmittel Wasserstoff eingesetzt wird.

7.  Verfahren nach Anspruch 6, <u>dadurch gekennzeichnet</u>, daß als Edelmetall Platin eingesetzt wird.

8.  Verfahren nach Anspruch 6, <u>dadurch gekennzeichnet</u>, daß als Edelmetall Palladium eingesetzt wird.

9.  Verfahren nach einem oder mehreren der Ansprüche 6 bis 8, <u>dadurch gekennzeichnet</u>, daß es sich um eine wäßrige Lösung des Edelmetalls handelt.

10. Verfahren nach einem oder mehreren der Ansprüche 6 bis 9, <u>dadurch gekennzeichnet</u>, daß als Trägermaterial Kieselgel ($SiO_2$) eingesetzt wird.

11. Verfahren nach einem oder mehreren der Ansprüche 6 bis 9, <u>dadurch gekennzeichnet</u>, daß als Trägermaterial Aluminiumoxid ($Al_2O_3$) eingesetzt wird.

12. Verfahren nach einem oder mehreren der Ansprüche 6 bis 9, <u>dadurch gekennzeichnet</u>, daß als Trägermaterial Zirkonoxid ($ZrO_2$) eingesetzt wird.

13. Verfahren nach einem oder mehreren der Ansprüche 6 bis 12, <u>dadurch gekennzeichnet</u>, daß das Trägermaterial in Form eines Pulvers eingesetzt wird.

14. Verfahren nach Anspruch 13, <u>dadurch gekennzeichnet</u>, daß das Pulver eine mittlere Partikelgröße von 50 bis 200 $\mu$m, eine BET-Oberfläche zwischen 250 und 600 $m^2$/g und ein Porenvolumen von 0,6 bis 1,2 ml/g aufweist.

15. Verfahren nach einem oder mehreren der Ansprüche 6 bis 14, <u>dadurch gekennzeichnet</u>, daß das Trägermaterial nach der Imprägnierung einer Trocknung unterzogen wird, durch die absorbiertes Wasser entfernt wird.

16. Verwendung von Suspensionsträgerkatalysatoren nach einem oder mehreren der Ansprüche 1 bis 5 oder hergestellt nach dem Verfahren der Ansprüche 6 bis 15 zur Dehalogenierung von $\alpha$-halogenierten Carbonsäuren.

17. Verwendung nach Anspruch 16, <u>dadurch gekennzeichnet</u>, daß es sich bei der Dehalogenierung um eine Dechlorierung handelt.

Abbildung 1:

## Abbildung 2:

**Europäisches Patentamt**

# EUROPÄISCHER RECHERCHENBERICHT

Nummer der Anmeldung

EP 98 10 2864

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | GB 2 211 756 A (SHELL INT RESEARCH)<br><br>* Beispiele *<br>--- | 1,3,5-7,<br>9,11,15 | B01J23/38<br>B01J23/42<br>C07C51/377 |
| X | US 5 350 727 A (TSURUMI KAZUNORI ET AL)<br><br>* Beispiele *<br>--- | 1-3,5-7,<br>9,13-15 | |
| X | US 4 082 699 A (PETROW HENRY G ET AL)<br><br>* Beispiele 10,11 *<br>--- | 1,3,6,7,<br>9-13,15 | |
| X | US 4 379 778 A (DALTON JR AUGUSTINE I ET AL)<br>* Spalte 4, Zeile 14 - Spalte 4, Zeile 19; Beispiele *<br>--- | 1,2,4,6,<br>8-13,15 | |
| A | DE 22 40 466 A (KNAPSACK AG)<br>* das ganze Dokument *<br>----- | | |

**RECHERCHIERTE SACHGEBIETE (Int.Cl.6)**

B01J
C07C

Der vorliegende Recherchenbericht wurde für alle Patentansprüche erstellt

| Recherchenort | Abschlußdatum der Recherche | Prüfer |
|---|---|---|
| MÜNCHEN | 19.März 1998 | Schwaller, J-M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................
& : Mitglied der gleichen Patentfamilie, übereinstimmendes Dokument

EPO FORM 1503 03.82 (P04C03)